# DEMANDE DE BREVET EUROPEEN

(11) **EP 4 717 219 A1**
(43) Date de publication de la demande: **01.04.2026**
(21) Numéro de dépôt: 24202394.3
(22) Date de dépôt: 25.09.2024
(51) Int. Cl.: A61B 34/10, A61B 17/70, A61B 17/88, A61D 1/00, G16H 50/50, A61B 34/00

(54) **IMPLANT OSSEUX EXPANSIBLE DE CHIRURGIE ORTHOPÉDIQUE VÉTÉRINAIRE, SYSTÈME ORTHOPÉDIQUE ET PROCÉDÉ DE FABRICATION DE L IMPLANT**

(71) Demandeur: IN LIFE VET SA, 1630 Bulle (CH)
(72) Inventeur: Lacaze, Guillaume, 1278 La Rippe (CH)
(74) Mandataire: Gsmart

(57) **Abrégé**

La présente invention concerne un système et une méthode de planification de chirurgie orthopédique vétérinaire, pour la restauration de volume et/ou géométrie d'un os, par une expansion entre une configuration repliée et une configuration déployée d'au moins un implant (1) osseux expansible comportant un corps s'étendant selon un axe longitudinal (L) entre une extrémité proximale (11) apte à coopérer avec un instrument d'implantation (A) pour tenir l'implant et une extrémité distale (12) destinée à être insérée en premier dans l'os, le système étant **caractérisé en ce** qu'il comprend au moins un implant (1) osseux expansible et au moins un instrument d'implantation (A) correspondant, sélectionnés parmi une pluralité d'implants (1) et d'instruments (A) disponibles grâce à des moyens informatiques (PC) comprenant une interface homme-machine et exécutant des instructions sur un processeur permettant :
- l'affichage d'informations relatives à au moins une classification standardisée des divers types de fractures identifiés, notamment les fractures de compression vertébrale ;
- la sélection d'au moins un type de fracture et d'au moins une information relative aux dimensions de l'os fracturé, puis
- l'affichage des implants (1) et instruments (A) du système qui sont utilisables pour la restauration envisagée et éventuellement de consignes ou conseils sur la procédure à suivre, par exemple avec un affichage de références des implants ou des divers systèmes utilisables pour chaque fracture identifiée dans la classification, puis
- la sélection d'un choix parmi ces propositions et conseils, par l'utilisateur via l'interface homme-machine, provoquant l'affichage d'instructions ou conseils suivants pour fournir une assistance à l'intervention ou sa planification, avec l'acquisition de données fournies par l'utilisateur sur l'intervention chirurgicale envisagée.

## Description

La présente demande se rapporte au domaine de la chirurgie, en particulier la chirurgie orthopédique vétérinaire et notamment du traitement d'une structure osseuse affaissée, par une restauration du volume (ou redressement) et/ou de la géométrie de cette structure osseuse. La présente demande concerne en particulier des instruments pour l'installation d'implants osseux expansibles permettant de réparer ou restaurer de structures osseuses lésées, notamment dans le rachis pour le traitement (souvent appelé « réduction ») des fractures de compression, notamment vertébrales (VCF pour l'anglais « Vertébral Compression Fracture »).

Dans ce domaine, le problème de la restauration du volume de structure osseuse qui s'est affaissée est bien connu et la littérature abonde de solutions utilisant des implants expansibles capables de passer d'une configuration repliée à une configuration déployée pour restaurer la hauteur de la structure osseuse, de préférence en combinaison avec une injection de ciment de substitution osseuse, dit ciment (ou ciment osseux). De nombreux ciments sont connus et ils présentent tous l'avantage d'être injectables à l'état liquide ou visqueux pendant une certaine durée, puis de durcir (par polymérisation) à l'intérieur de la structure osseuse pour la stabiliser.

Des problèmes majeurs dans ce domaine concernent l'expansion de l'implant pour restaurer une hauteur au tissu osseux lésé et la fuite de ciment, mais également l'installation des implants dans les os, notamment les vertèbres. En effet, il est nécessaire de disposer d'instruments facilitant l'implantation et réduisant autant que possible la durée et la lourdeur de la tâche pour les praticiens.

D'autres problèmes récurrents en chirurgie orthopédique concernent l'invasivité (c'est-à-dire le but de faire une incision et des lésions les moins étendues possibles) mais également le ratio de déploiement afin d'obtenir un implant déployé qui comble le plus grand volume possible tout en ayant été introduit par un passage le plus petit possible. D'autre part, ce ratio de déploiement va impacter la répartition des forces pour redresser les vertèbres : si on est trop déformable, la pression d'injection du ciment va plutôt déformer la poche au lieu de restaurer la hauteur.

Dans ce contexte, on comprend qu'il persiste dans le domaine de nombreux problèmes techniques liés aux implants et qui s'accompagnent de problèmes concernant les instruments d'implantation qui sont généralement trop nombreux et complexes pour répondre aux problèmes majeurs de l'invasivité et de la facilité et la durée de l'intervention chirurgicale. De plus, le choix des implants en fonction du type de fracture reste difficile et des solutions offrant une approche pragmatique pour planifier les interventions chirurgicales font encore défaut dans le domaine.

Dans ce contexte, un but de la présente invention est de pallier au moins certains inconvénients de l'art antérieur en proposant un système d'implants orthopédiques, pour la restauration de structure osseuses affaissées.

Ce but est atteint par un système de chirurgie orthopédique vétérinaire, pour la restauration de volume et/ou géométrie d'un os, par une expansion entre une configuration repliée et une configuration déployée d'au moins un implant osseux expansible comportant un corps s'étendant selon un axe longitudinal entre une extrémité proximale apte à coopérer avec un instrument d'implantation pour tenir l'implant et une extrémité distale destinée à être insérée en premier dans l'os, le système étant caractérisé en ce qu'il comprend au moins un implant osseux expansible et au moins un instrument d'implantation correspondant, sélectionnés parmi une pluralité d'implants et d'instruments disponibles grâce à des moyens informatiques comprenant une interface homme-machine et exécutant des instructions sur un processeur permettant :
- l'affichage d'informations relatives à au moins une classification standardisée des divers types de fractures identifiés, notamment les fractures de compression vertébrale ;
- la sélection d'au moins un type de fracture et d'au moins une information relative aux dimensions de l'os fracturé, puis
- l'affichage des implants et instruments du système qui sont utilisables pour la restauration envisagée et éventuellement de consignes ou conseils sur la procédure à suivre, par exemple avec un affichage de références des implants ou des divers systèmes utilisables pour chaque fracture identifiée dans la classification, puis
- la sélection d'un choix parmi ces propositions et conseils, par l'utilisateur via l'interface homme-machine, provoquant l'affichage d'instructions ou conseils suivants pour fournir une assistance à l'intervention ou sa planification, avec l'acquisition de données fournies par l'utilisateur sur l'intervention chirurgicale envisagée.

Selon une autre particularité, ladite sélection du type de fracture dans la classification est réalisée soit par l'utilisateur soit de manière automatique par les moyens informatiques grâce à un entrainement sur des bases de données de fractures pour leur classification automatique à partir d'informations techniques et/ou d'images fournies par l'utilisateur et/ou acquises au préalable par les moyens informatique, puis leur mise en relation avec ladite classification standardisée des fractures, la sélection automatique étant de préférence validable ou modifiable par l'utilisateur.

Selon une autre particularité, l'un des implants sélectionnables est un implant osseux expansible de chirurgie orthopédique vétérinaire pour la restauration de volume et/ou géométrie d'un os, par une expansion entre une configuration repliée et une configuration déployée, ledit implant comportant un corps creux s'étendant selon un axe longitudinal entre une extrémité proximale apte à coopérer avec un instrument d'implantation pour tenir l'implant et une extrémité distale destinée à être insérée en premier dans l'os, et qui est caractérisé en ce que :
- la paroi dudit corps creux est formée par une feuille en alliage biocompatible de métaux, refermée sur elle-même de manière étanche, entre lesdites extrémités proximale et distale ;
- ladite feuille présente, au moins dans la configuration repliée, une pluralité de paires de plis, chacune des paires comportant un pli antiforme, dit convexe, et un pli synforme, dit concave, lesdits plis étant couchés les uns sur les autres en configuration repliée de sorte que les surfaces présentes entre chacun desdits plis convexe et concave soient enroulées autour de l'axe longitudinal,
- ladite extrémité proximale comporte une bague solidarisée, de manière étanche, aux plis couchés et enroulés de ladite feuille sur toute la périphérie de l'extrémité distale, l'ouverture traversant la bague fournissant une entrée vers l'intérieur du corps creux de l'implant,
- ladite extrémité distale comporte un culot fermant l'extrémité distale et solidarisé, de manière étanche, aux plis couchés et enroulés de ladite feuille sur toute la périphérie de l'extrémité distale dudit corps creux
- ladite feuille étant déformable plastiquement pour permettre l'expansion de l'implant de la configuration repliée à la configuration déployée, lors de l'injection d'un fluide à l'intérieur de l'implant.

Selon une autre particularité, l'un des implants sélectionnables est un implant osseux expansible de chirurgie orthopédique vétérinaire pour la restauration de volume et/ou géométrie d'un os, par une expansion entre une configuration repliée et une configuration déployée, ledit implant s'étendant selon un axe longitudinal entre une extrémité proximale apte à coopérer avec un instrument d'implantation pour tenir l'implant et une extrémité distale destinée à être insérée en premier dans l'os, au moins deux faces de l'implant, par exemple supérieure et inférieure, comportant chacune un plateau pour le contact avec les tissus osseux, chacun des plateaux comportant une portion centrale reliée, par l'intermédiaire d'au moins une charnière, à au moins une paire de bras de support orientés chacun dans des directions opposées au sein de chaque paire, un bras de chaque paire étant relié par une charnière à l'extrémité distale tandis que l'autre bras est relié par une charnière à l'extrémité proximale, l'implant étant apte à recevoir ou comportant un axe central s'étendant au travers d'un manchon de coulissement à l'extrémité proximale jusqu'à une bague ou une douille de traction à l'extrémité distale où il est apte à transmettre une traction, lors de son actionnement par un instrument, sur l'extrémité distale pour permettre de la rapprocher de l'extrémité proximale, en engendrant le pivotement des bras de support provoquant l'éloignement des plateaux l'un par rapport à l'autre et, par conséquent, l'expansion de l'implant entre la configuration repliée et la configuration déployée.

Selon une autre particularité, l'implant est également caractérisé en ce que :
- au moins deux autres faces de l'implant, entre celles comportant les plateaux, sont couvertes d'au moins une feuille par face, en alliage biocompatible de métaux, et solidarisée de manière étanche aux portions centrales sous les plateaux, à des faces latérales des bras et à des faces latérales de l'extrémité proximale et de l'extrémité distale,
- ladite feuille est déformable plastiquement pour permettre l'expansion de l'implant et présente, au moins dans la configuration repliée, une pluralité de plis antiformes, dit convexes, et synformes, dit concaves, lesdits plis étant couchés les uns sur les autres en configuration repliée, la surface totale de ladite feuille étant supérieure ou égale à la surface latérale de l'implant en configuration déployée de façon à former un compartiment étanche apte à accueillir un fluide à l'intérieur de la cavité obtenue par l'expansion de l'implant.

Selon une autre particularité, ledit implant comporte une enveloppe renfermant ledit implant depuis l'extrémité proximale jusqu'à l'extrémité distale et en ce que :
- ladite enveloppe est formée par une feuille en alliage biocompatible de métaux, refermée sur elle-même de manière étanche ;
- ladite feuille présente, au moins dans la configuration repliée, une pluralité de paires de plis, chacune des paires comportant un pli antiforme, dit convexe, et un pli synforme, dit concave, lesdits plis étant couchés les uns sur les autres en configuration repliée de sorte que les surfaces présentes entre chacun desdits plis convexes et concaves soient enroulées autour de l'axe longitudinal ;
- ladite extrémité proximale se prolonge par un manchon d'étanchéité solidarisé de manière étanche, aux plis couchés et enroulés de ladite feuille sur toute la périphérie de l'extrémité proximale ;
- ladite extrémité distale se prolonge par une douille solidarisé, de manière étanche, aux plis couchés et enroulés de ladite feuille sur toute la périphérie de l'extrémité distale dudit implant ;
- ladite feuille est déformable plastiquement pour permettre l'expansion de l'implant de la configuration repliée à la configuration déployée en formant une enveloppe étanche enfermant l'implant et permettant d'éviter les fuites lors de l'injection d'un fluide à l'intérieur de l'implant et l'enveloppe.

Selon une autre particularité, l'un des implants sélectionnables est un implant osseux expansible de chirurgie orthopédique vétérinaire pour la restauration de volume et/ou géométrie d'un os, par une expansion entre une configuration repliée et une configuration déployée, ledit implant comportant un axe central et s'étendant selon un axe longitudinal entre une extrémité proximale apte à coopérer avec un instrument d'implantation pour tenir l'implant et une extrémité distale destinée à être insérée en premier dans l'os, au moins deux faces, par exemple supérieure et inférieure, de l'implant comportant chacune au moins un plateau pour le contact avec les tissus osseux, chacun des plateaux étant supportés par au moins deux bras de support chacun, par l'intermédiaire d'une charnière sur l'axe central et d'une charnière sous le plateau respectif de chacun desdits bras de support, caractérisé en ce que :
- une douille ou bague d'expansion disposée dans le même axe que ledit axe central ;
- au moins deux bras d'expansion comportent chacun une charnière les reliant à l'une des extrémités de l'un des plateaux et une charnière les reliant à ladite douille ou bague d'expansion ;
- ladite douille ou bague d'expansion et l'extrémité proximale de l'axe central sont aptes à coopérer, respectivement ou inversement, avec un tube creux de préhension de l'implant d'un instrument d'implantation et avec une tige d'expansion dudit instrument ;
- ladite tige d'expansion est apte à coulisser à l'intérieur dudit tube creux, de façon à permettre de provoquer un éloignement entre ladite douille et ledit axe central, exerçant une traction sur les plateaux, par l'intermédiaire des bras d'expansion, ce qui engendre un pivotement desdits bras de support provoquant l'éloignement des plateaux par rapport à l'axe central, de manière à résulter en une expansion contrôlée de l'implant entre ladite configuration repliée et ladite configuration déployée.

Selon une autre particularité, l'implant comporte une enveloppe renfermant ledit implant depuis l'extrémité proximale jusqu'à l'extrémité distale et en ce que :
- ladite enveloppe est formée par une feuille en alliage biocompatible de métaux, refermée sur elle-même de manière étanche ;
- ladite feuille présente, au moins dans la configuration repliée, une pluralité de paires de plis, chacune des paires comportant un pli antiforme, dit convexe, et un pli synforme, dit concave, lesdits plis étant couchés les uns sur les autres en configuration repliée de sorte que les surfaces présentes entre chacun desdits plis convexes et concaves soient enroulées autour de l'axe longitudinal ;
- ladite extrémité proximale se prolonge par un manchon d'étanchéité solidarisé de manière étanche, aux plis couchés et enroulés de ladite feuille sur toute la périphérie de l'extrémité proximale ;
- ladite extrémité distale se prolonge par une douille solidarisé, de manière étanche, aux plis couchés et enroulés de ladite feuille sur toute la périphérie de l'extrémité distale dudit implant ;
- ladite feuille est déformable plastiquement pour permettre l'expansion de l'implant de la configuration repliée à la configuration déployée en formant une enveloppe étanche enfermant l'implant et permettant d'éviter les fuites lors de l'injection d'un fluide à l'intérieur de l'implant et l'enveloppe.

Un autre but de la présente demande est de pallier au moins une partie des inconvénients de l'art antérieur en proposer une méthode de planification d'intervention chirurgicale facile à utiliser et permettant une stabilisation efficace des tissus osseux ou même une assistance à l'intervention et/ou au suivi de la guérison.

Ce but est atteint par une méthode de planification d'implantation de système d'implants selon l'une des revendications précédentes, pour la restauration de volume et/ou géométrie d'un os, par une expansion entre une configuration repliée et une configuration déployée, caractérisé en ce qu'elle comporte une sélection, automatique et/ou par le praticien réalisant l'implantation, d'au moins un type de fracture identifié dans au moins une classification standardisée, via des moyens informatiques et présenté audit praticien, puis l'accès, suite à cette sélection, à au moins un système d'implants compatible avec ledit type de fracture et à au moins un protocole opératoire comprenant une succession d'étapes à réaliser en fonction de ladite sélection.

Selon une autre particularité, la méthode est mise en oeuvre par des moyens informatiques exécutant des instructions sur un processeur permettant :
- l'affichage d'informations relatives à au moins une classification des types de fractures identifiées en traumatologie ;
- la sélection d'au moins un type de fracture et d'au moins une information relative aux dimensions de l'os fracturé, puis
- l'affichage de consignes ou conseils sur la procédure à suivre et sur les divers systèmes à utiliser pour la procédure, par exemple avec un affichage de références des implants ou des divers systèmes utilisables pour chaque fracture identifiée dans la classification, puis
- la sélection d'un choix parmi ces conseils, provoquant l'affichage d'instructions ou conseils suivants pour fournir ladite assistance étape par étape.

Selon une autre particularité, l'affichage de consignes ou conseils comprend l'affichage de fonctions sélectionnables pour réaliser un choix parmi des actes à effectuer pour continuer la procédure et/ou parmi des implants à sélectionner en fonction du protocole opératoire choisi en fonction du type de fracture identifié d'après ladite classification, permettant au praticien de valider un choix et éventuellement proposer des options prédéfinies dans le protocole validé, comme par exemple l'acquisition de radiographies à la fin de certaines étapes mises en oeuvre.

Selon une autre particularité, lesdites fonctions sélectionnables incluent des fonctions permettant au praticien de modifier ledit protocole et générer l'affichage d'une fenêtre de modification du protocole, soit pour une sélection d'un protocole parmi d'autres protocoles possibles pour ledit type de fracture identifié d'après ladite classification, soit pour la création d'un protocole opératoire personnalisé pour la fracture en cours de réparation.

Selon une autre particularité, lesdites fonctions sélectionnables permettent un enregistrement des opérations et protocoles opératoires, notamment personnalisés, utilisés par les différents praticiens et leurs associations aux fractures, avec un éventuel enregistrement de protocoles personnalisés créées par les praticiens, afin de les proposer ultérieurement.

D'autres particularités et avantages de la présente invention apparaîtront plus clairement à la lecture de la description de divers modes de réalisation ci-après, faite en référence aux dessins annexés, dans lesquels :

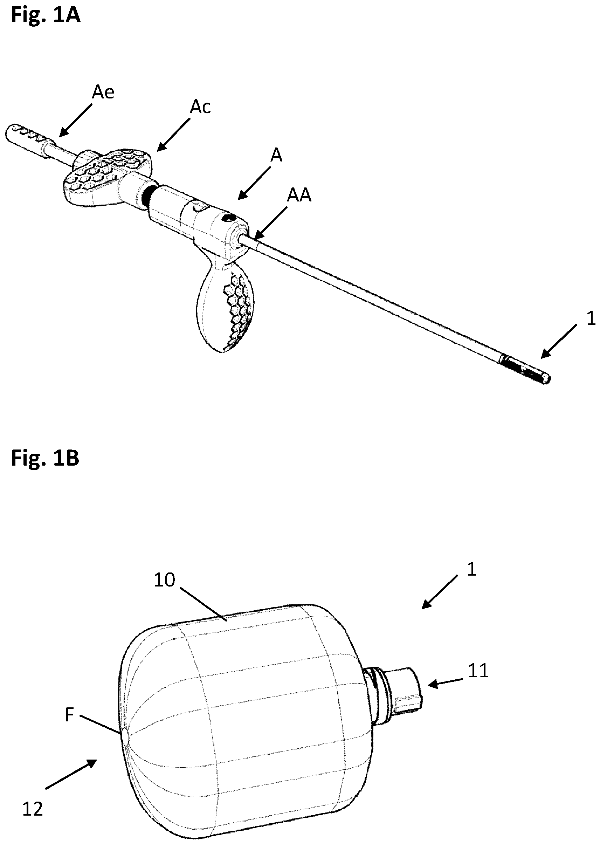

La figure 1A représente une vue en perspective d'un instrument d'implantation portant un implant osseux expansible en configuration repliée, selon certains modes de réalisation, et la figure 1B représente ce même implant en configuration déployée ;

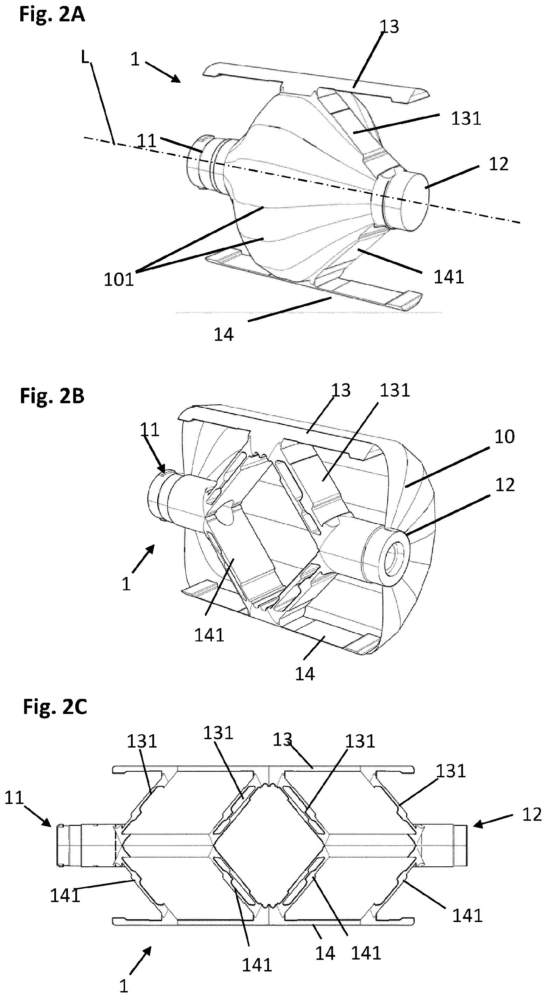

La figure 2A représente une vue en perspective d'un implant expansible selon certains modes de réalisation, la figure 2B représente une vue en perspective avec une partie en couple d'un implant expansible selon d'autres modes de réalisation et la figure 2C représente une vue de profil d'un implant expansible selon encore d'autres modes de réalisation ;

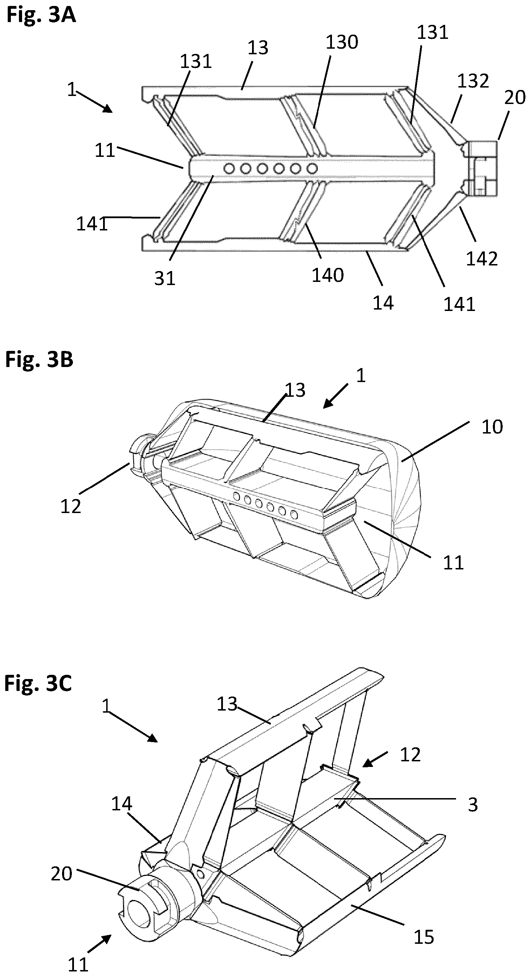

La figure 3A représente une vue de profil d'un implant expansible selon certains modes de réalisation, la figure 3B représente une vue en perspective avec une partie en couple d'un implant expansible selon d'autres modes de réalisation et la figure 3C représente une vue de profil d'un implant expansible selon encore d'autres modes de réalisation ;

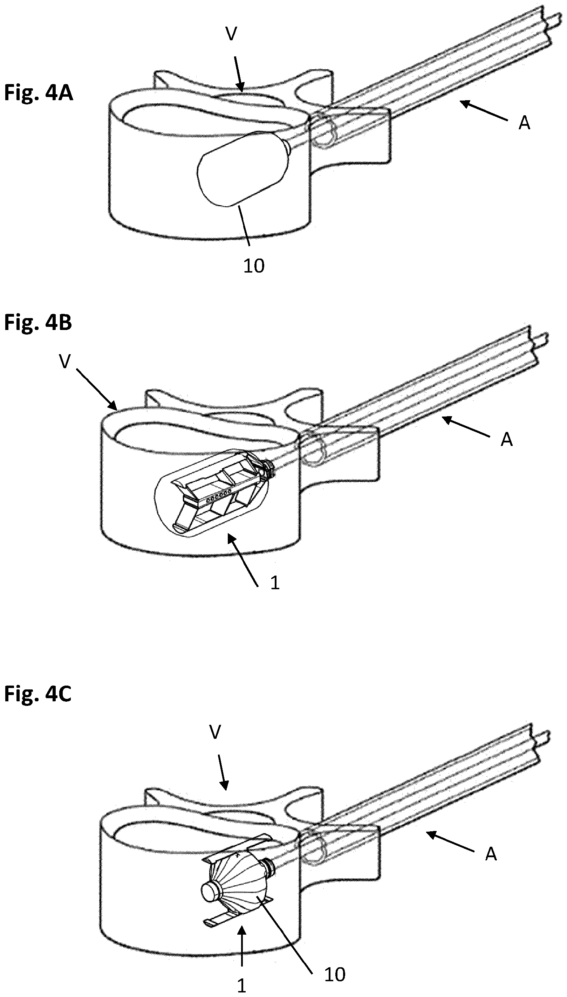

La figure 4A représente une vue en perspective de l'instrumentation d'implantation d'implant expansible comprenant un porte-implant un instrument d'injection de ciment et un instrument de préhension, la figure 4B représente détail de la partie circulaire indiquée sur
Les figures 4A, 4B et 4C représentent des vues en perspective de vertèbres dans lesquelles sont insérés un implant expansible selon des modes de réalisation différents ;

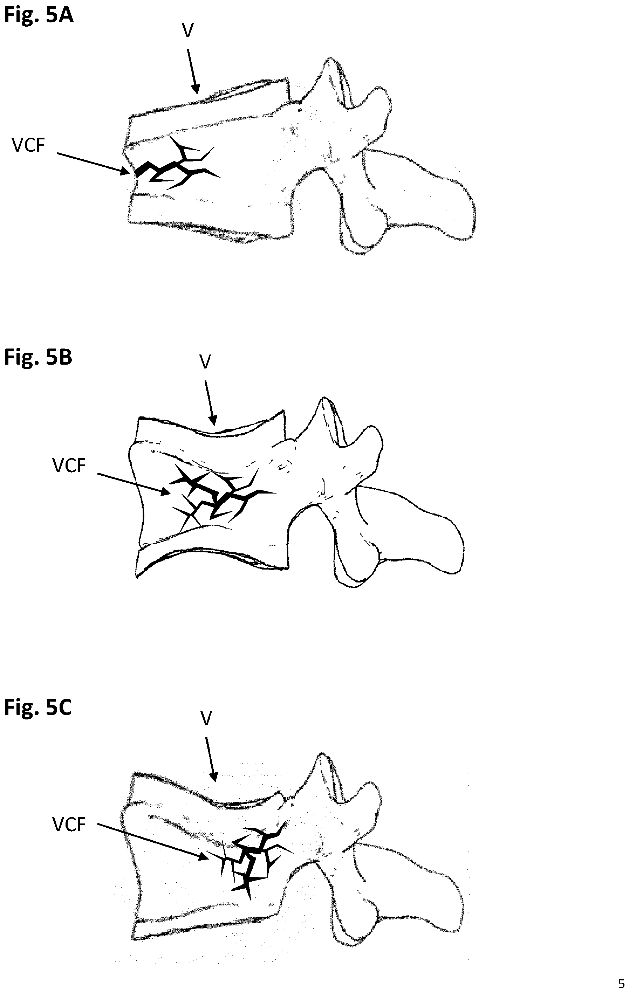

Les figures 5A, 5B et 5C représentent des vues de profil de vertèbres ayant subi des fractures de compression vertébrale (VCF) respectivement au niveau antérieur médian et postérieur ;

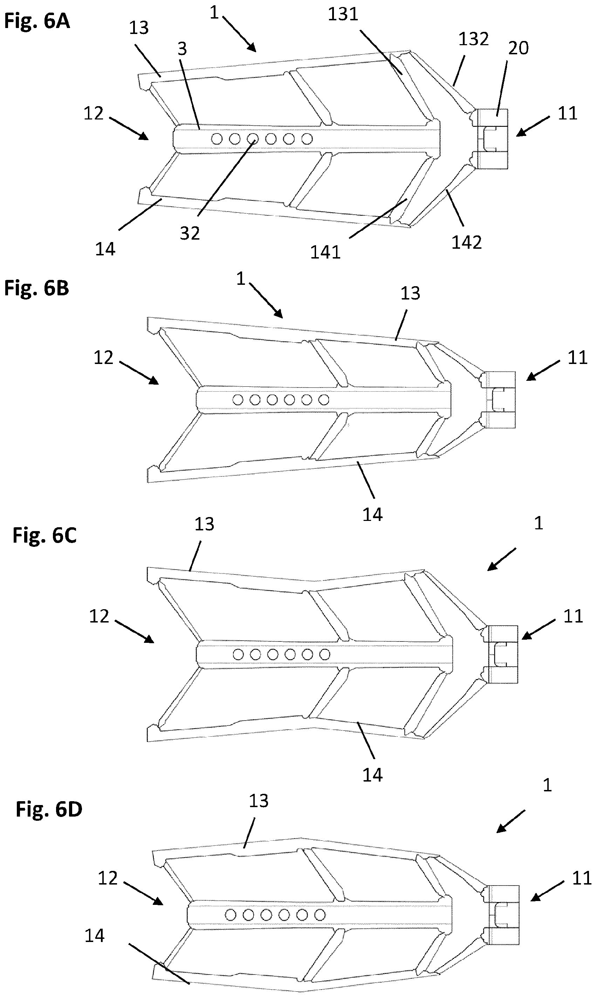

Les figures 6A, 6B, 6C et 5CD représentent des vues de profil d'implants expansibles du même type mais à géométrie différentes pour l'adaptation aux fractures de vertèbres notamment celles des figures 5A ,5B et 5C ;

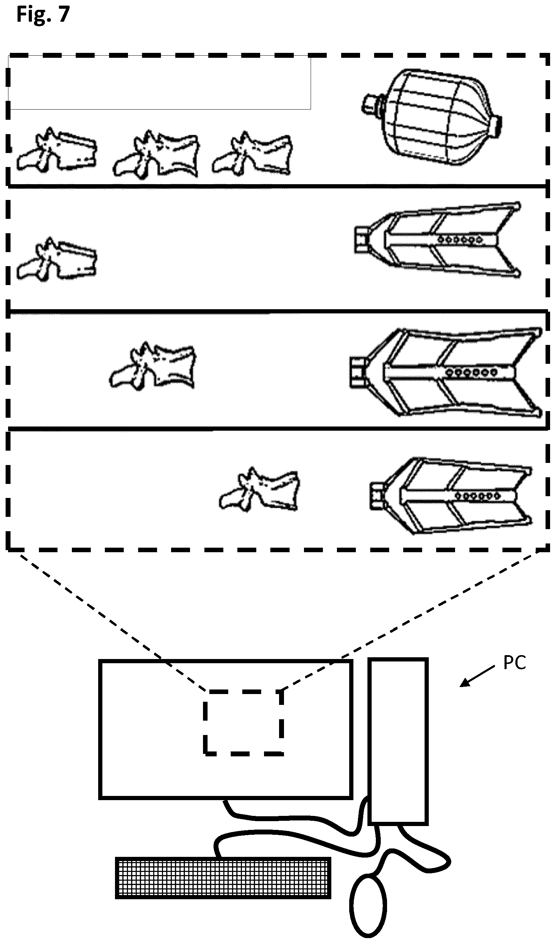

La figure 7 représente une vue schématique du système de planification d'intervention utilisant un affichage de la classification de fracture et des implants utilisables selon les cas.

La présente demande concerne système de chirurgie orthopédique vétérinaire pour le traitement d'os fracturé et de tissus osseux en général et un procédé de planification d'intervention chirurgicale et/ou d'assistance à l'intervention. L'implant osseux est de préférence un implant Rachidien, et en particulier vertébral ou même en fait intravertébral, mais d'autres utilisations sont envisageables ailleurs dans le rachis (épines intervertébrales) ou dans d'autres structures osseuses où il est nécessaire de combler un espace laissé résultant d'une fracture (dont les causes peuvent être diverses, même si elles impliquent généralement une diminution de densité osseuse). Ainsi, les fractures vertébrales de compression (VCF pour l'anglais Vertébral Compression Fracture) sont une application favorite mais ne sont pas les seules à pouvoir être traitées grâce à la présente invention et l'homme de métier appréciera les possibilités offertes sans nécessiter plus de détails ici. Comme autre os, on peut citer le fémur ou l'humérus (tête) par exemple en cas de risque d'effondrement. La présente demande concerne un implant et un système de chirurgie orthopédique vétérinaire pour le traitement d'os fracturé et de tissus osseux en général, ainsi qu'un procédé de fabrication de l'implant. L'implant osseux est de préférence un implant Rachidien, et en particulier vertébral ou même en fait intravertébral, mais d'autres utilisations sont envisageables ailleurs dans le rachis (épines intervertébrales) ou dans d'autres structures osseuses où il est nécessaire de combler un espace laissé résultant d'une fracture (dont les causes peuvent être diverses, même si elles impliquent généralement une diminution de densité osseuse). Ainsi, les fractures vertébrales de compression (VCF pour l'anglais Vertébral Compression Fracture) sont une application favorite mais ne sont pas les seules à pouvoir être traitées grâce à la présente invention et l'homme de métier appréciera les possibilités offertes sans nécessiter plus de détails ici. Comme autre os, on peut citer le fémur ou l'humérus (tête) par exemple en cas de risque d'effondrement. Dans le domaine vétérinaire, il est connu que les animaux ont des densités osseuses parfois très différentes de l'homme et surtout très variables selon les espèces et même selon les races ou animaux au sein d'une même espèces, notamment pour les chiens dont les propriétés physiques varient énormément d'une race à l'autre. Par exemple, les chiens de type teckel ou similaires ont des vertèbres longues (hautes) mais peu larges comparé aux autres espèces. Il est donc utile de disposer d'implants expansibles qui permettent une forte expansion en hauteur tout en ayant une longueur réduite et il est clairement nécessaire de tenir compte des différences de formes et dimensions des os des différentes espèces pour adapter la thérapie efficacement avec des implants adaptés. De plus, certaines espèces comme le chat ont une corticale osseuse très rigide mais du tissu spongieux plus souple que d'autres espèces. Il est donc nécessaire de prendre également la nature des tissus osseux. Enfin, un autre exemple notable concerne les chevaux qui ont des os, notamment les vertèbres, avec une forme anatomique particulière et qui supportent parfois une charge importante. Selon l'activité (par exemple sportive) et la morphologie, la densité osseuse varie et les implants doivent être adaptés pour permettre leur expansion mais aussi supporter les charges. Ainsi, pour un cheval, il peut être nécessaire d'avoir des implants comprenant plus de bras de support de charge (minimum 3 ou 4) que pour d'autres espèces (où 2 bras support suffisent parfois). En l'absence de bras de support, il est alors nécessaire que l'implant ait une résistance mécanique suffisante, grâce à une capacité à supporter une pression de ciment supérieure à d'autres cas. Par ailleurs, certains modes de réalisation comportant plus de deux plateaux peuvent notamment servir plus efficacement au traitement d'os longs de ce type en répartissant les forces d'expansion sur plus deux surfaces, ce qui fournit une meilleure stabilité quel que soit le type d'os.

Certains modes de réalisation prévoient l'injection d'un fluide (e.g., « ciment osseux », généralement à base d'un polymère comme le PMMA par exemple et bien connu de l'homme de métier, de sorte qu'aucun détail sur le ciment ne sera fourni ici). Ainsi, l'implant une fois positionné peut notamment être stabilisé grâce une telle injection de ciment. Cependant, comme les fuites de ciment restent un problème majeur dans le domaine, divers modes de réalisation proposent de contenir le ciment dans une enveloppe étanche dont le volume après injection peut être maîtrisé grâce à la structure et le matériau de l'enveloppe, en fonction de la pression injectée (et de la configuration des tissus osseux préférentiellement évaluée à l'avance, comme généralement pratiqué dans le domaine). L'étanchéité est bien entendu relative et ce terme n'est pas limitatif non plus, puisque le niveau d'étanchéité est en fait adapté à la viscosité du ciment au moment où on l'injecte. Certains modes de réalisation permettent en particulier un gonflement homothétique de l'enveloppe grâce à la souplesse (relative) de la feuille (10) de matériau métallique biocompatible. Ce matériau est en général un alliage de titane obtenu sous forme de feuille très fine, de préférence par lamination pour un état de surface et une épaisseur maîtrisés, notamment une épaisseur comprise entre 3 et 100 microns, généralement entre 6 et 50 et de préférence 10 et 30 microns. Cette feuille est capable de déformation plastique réversible un nombre de fois satisfaisant pour l'application visée puisqu'elle offre notamment la possibilité de rétracter l'enveloppe formée par la feuille en cas de problème (biocompatibilité et résistance au déchirement). En effet, en général, le pilotage du dosage de ciment permet de surveiller les quinze minutes de polymérisation pendant lesquelles il est possible de rétracter l'enveloppe et aspirer le ciment. D'autre part, par l'injection de ciment, le gonflement de l'enveloppe, l'implant remplit les espaces dans les tissus lésés en fonction des forces de compression et de résistance osseuse par rapport à la pression hydraulique fournie lors de l'injection de ciment. A partir d'une telle feuille, il est nécessaire d'obtenir une structure fermée, ce qui impose déjà de refermer la feuille sur elle-même et de la verrouiller en position. Pour cela, une soudure (ou un collage ou une brasure, ces termes n'étant pas limitatifs ici) peut être réalisée entre deux bords qui se superposent ou sur des bords avec des revers enchevêtrés, pour faciliter et solidifier la soudure. Certains modes de réalisation prévoient donc une fermeture par soudure depuis l'extérieur, simplifié et plus solide grâce à la superposition de couches au niveau de ces plis complémentaires.

Le terme « solidarisé » signifie ici que deux éléments sont rendus solidaires, soit de manière permanente (ou quasi-permanente) mais également parfois qu'une liaison est réalisée pour l'actionnement d'un élément par un autre. Ainsi, un vissage ou une coopération de forme pour verrouiller provisoirement les éléments entre sont couverts par ce terme non limitatif.

Les termes bague, manchon, ou tube désignent des structures creuses telles que des anneaux, des conduits ou des tuyaux mais de façon non limitative, notamment avec des formes diverses (à l'intérieur comme à l'extérieur) bien que la forme cylindrique soit préférée. Le terme canal est en revanche de préférence utilisé ici pour désigner un passage plutôt que l'élément qui le contient et le terme ouverture désigne ici le fait qu'un élément soit ouvert et apte à être traversé en débouchant dans une autre structure ou un autre élément. Généralement les termes manchon et tubes ou conduits désignent des éléments plus longs que les bagues ou anneaux, mais leur utilisation ici n'est pas limitative non plus. D'autre part, les termes douille ou culot désignent également des structures creuses qui sont ouvertes à une extrémité mais fermées à l'autre extrémité, comme des bouchons, des fermetures, des constrictions ou étranglement et ces termes sont utilisés indifféremment sans limitation quelconque.

On notera que l'instrument d'injection de fluide (Ac) peut être muni de moyens de contrôler la pression et/ou la quantité injectée (un manomètre ou au moins des graduations par exemple) et de savoir quel est le volume résultant, afin de contrôler efficacement l'expansion dans les tissus osseux. Avantageusement, des moyens de contrôle de l'air injecté peuvent être présents pour adapter l'injection de ciment en fonction de l'évacuation de l'air dans les tubes creux ou canules des instruments (l'air s'échappant généralement facilement depuis l'implant vers l'instrument grâce au jeu entre les pièces (tiges et tubes ou canules) de l'instrument).

Enfin, on comprend que l'instrumentation proposée dans la présente demande dans certains modes de réalisation, en utilisant un porte-implant (ou ancillaire) relativement classique pour tenir l'implant et l'introduire dans les tissus osseux, mais également moins classique pour l'expandre dans les tissus osseux, présente également l'avantage de pouvoir réaliser toutes les étapes d'implantation et de stabilisation avec un seul instrument et en une opération continue. En effet, l'ancillaire avec un tube creux d'acheminement du ciment au travers du tube qui retient le ciment, permet de disposer d'un instrument permettant d'effectuer l'opération chirurgicale rapidement et efficacement. Après le perçage, on introduit l'implant et sans retirer l'instrument, on peut gonfler l'enveloppe avec le ciment et ensuite retirer l'outil avant, pendant ou même après la polymérisation du ciment (par exemple à l'aide d'un mécanisme de coupe du ciment dur lors d'une rotation de l'instrument). Le temps d l'opération chirurgicale est bien entendu nettement diminué mais également la stabilité de l'implant qui n'est lâché à aucun moment tant qu'il n'est pas stabilisé par l'injection de ciment remplissant tous les volumes libres autour, contrairement à certaines solutions de l'art antérieur.

D'une manière générale, la présente demande concerne La présente demande concerne un système de chirurgie orthopédique vétérinaire, pour la restauration de volume et/ou géométrie d'un os, par une expansion entre une configuration repliée et une configuration déployée d'au moins un implant (1) osseux expansible comportant un corps s'étendant selon un axe longitudinal (L) entre une extrémité proximale (11) apte à coopérer avec un instrument d'implantation (A) pour tenir l'implant et une extrémité distale (12) destinée à être insérée en premier dans l'os, le système étant caractérisé en ce qu'il comprend au moins un implant (1) osseux expansible et au moins un instrument d'implantation (A) correspondant, sélectionnés parmi une pluralité d'implants (1) et d'instruments (A) disponibles grâce à des moyens informatiques (PC) comprenant une interface homme-machine et exécutant des instructions sur un processeur permettant :
- l'affichage d'informations relatives à au moins une classification standardisée des divers types de fractures identifiés, notamment les fractures de compression vertébrale ;
- la sélection d'au moins un type de fracture et d'au moins une information relative aux dimensions de l'os fracturé, puis
- l'affichage des implants (1) et instruments (A) du système qui sont utilisables pour la restauration envisagée et éventuellement de consignes ou conseils sur la procédure à suivre, par exemple avec un affichage de références des implants ou des divers systèmes utilisables pour chaque fracture identifiée dans la classification, puis
- la sélection d'un choix parmi ces propositions et conseils, par l'utilisateur via l'interface homme-machine, provoquant l'affichage d'instructions ou conseils suivants pour fournir une assistance à l'intervention ou sa planification, avec l'acquisition de données fournies par l'utilisateur sur l'intervention chirurgicale envisagée.

Dans certains modes de réalisation, ladite sélection du type de fracture dans la classification est réalisée soit par l'utilisateur soit de manière automatique par les moyens informatiques (PC) grâce à un entrainement sur des bases de données de fractures pour leur classification automatique à partir d'informations techniques et/ou d'images fournies par l'utilisateur et/ou acquises au préalable par les moyens informatique, puis leur mise en relation avec ladite classification standardisée des fractures, la sélection automatique étant de préférence validable ou modifiable par l'utilisateur.

Dans certains modes de réalisation, l'un des implants sélectionnables est un implant (1) osseux expansible de chirurgie orthopédique vétérinaire pour la restauration de volume et/ou géométrie d'un os, par une expansion entre une configuration repliée et une configuration déployée, ledit implant comportant un corps creux s'étendant selon un axe longitudinal (L) entre une extrémité proximale (11) apte à coopérer avec un instrument d'implantation (A) pour tenir l'implant et une extrémité distale (12) destinée à être insérée en premier dans l'os, et qui est caractérisé en ce que :
- la paroi dudit corps creux est formée par une feuille (10) en alliage biocompatible de métaux, refermée sur elle-même de manière étanche, entre lesdites extrémités proximale (11) et distale (12) ;
- ladite feuille (10) présente, au moins dans la configuration repliée, une pluralité de paires de plis, chacune des paires comportant un pli antiforme (101), dit convexe, et un pli synforme (102), dit concave, lesdits plis étant couchés les uns sur les autres en configuration repliée de sorte que les surfaces présentes entre chacun desdits plis convexe et concave soient enroulées autour de l'axe longitudinal (L),
- ladite extrémité proximale (11) comporte une bague solidarisée, de manière étanche, aux plis couchés et enroulés de ladite feuille (10) sur toute la périphérie de l'extrémité distale (11), l'ouverture traversant la bague fournissant une entrée vers l'intérieur du corps creux de l'implant (1),
- ladite extrémité distale (12) comporte un culot fermant l'extrémité distale (12) et solidarisé, de manière étanche, aux plis couchés et enroulés de ladite feuille (10) sur toute la périphérie de l'extrémité distale dudit corps creux
- ladite feuille (10) étant déformable plastiquement pour permettre l'expansion de l'implant de la configuration repliée à la configuration déployée, lors de l'injection d'un fluide à l'intérieur de l'implant (1).

Dans certains modes de réalisation, l'un des implants sélectionnables est un implant (1) osseux expansible de chirurgie orthopédique vétérinaire pour la restauration de volume et/ou géométrie d'un os, par une expansion entre une configuration repliée et une configuration déployée, ledit implant s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces de l'implant, par exemple supérieure et inférieure, comportant chacune un plateau (13, 14) pour le contact avec les tissus osseux, chacun des plateaux comportant une portion centrale (130, 140) reliée, par l'intermédiaire d'au moins une charnière, à au moins une paire de bras de support (131, 141) orientés chacun dans des directions opposées au sein de chaque paire, un bras de chaque paire étant relié par une charnière à l'extrémité distale (11) tandis que l'autre bras est relié par une charnière à l'extrémité proximale (12), l'implant (1) étant apte à recevoir ou comportant un axe (3) central s'étendant au travers d'un manchon de coulissement à l'extrémité proximale (11) jusqu'à une bague ou une douille de traction à l'extrémité distale (12) où il est apte à transmettre une traction, lors de son actionnement par un instrument (A), sur l'extrémité distale (12) pour permettre de la rapprocher de l'extrémité proximale (11), en engendrant le pivotement des bras de support (131, 141) provoquant l'éloignement des plateaux (13, 14) l'un par rapport à l'autre et, par conséquent, l'expansion de l'implant entre la configuration repliée et la configuration déployée.

Dans certains de ces modes de réalisation, l'implant (1) est également caractérisé en ce que :
- au moins deux autres faces de l'implant, entre celles comportant les plateaux, sont couvertes d'au moins une feuille (10) par face, en alliage biocompatible de métaux, et solidarisée de manière étanche aux portions centrales (130, 140) sous les plateaux, à des faces latérales des bras (131, 141) et à des faces latérales de l'extrémité proximale (11) et de l'extrémité distale (12),
- ladite feuille (10) est déformable plastiquement pour permettre l'expansion de l'implant et présente, au moins dans la configuration repliée, une pluralité de plis antiformes (101), dit convexes, et synformes (102), dit concaves, lesdits plis étant couchés les uns sur les autres en configuration repliée, la surface totale de ladite feuille étant supérieure ou égale à la surface latérale de l'implant en configuration déployée de façon à former un compartiment étanche apte à accueillir un fluide à l'intérieur de la cavité obtenue par l'expansion de l'implant.

Dans certains modes de réalisation, ledit implant (1) comporte une enveloppe renfermant ledit implant depuis l'extrémité proximale (11) jusqu'à l'extrémité distale (12) et en ce que :
- ladite enveloppe est formée par une feuille (10) en alliage biocompatible de métaux, refermée sur elle-même de manière étanche ;
- ladite feuille (10) présente, au moins dans la configuration repliée, une pluralité de paires de plis, chacune des paires comportant un pli antiforme (101), dit convexe, et un pli synforme (102), dit concave, lesdits plis étant couchés les uns sur les autres en configuration repliée de sorte que les surfaces présentes entre chacun desdits plis convexes et concaves soient enroulées autour de l'axe longitudinal (L) ;
- ladite extrémité proximale (11) se prolonge par un manchon d'étanchéité solidarisé de manière étanche, aux plis couchés et enroulés de ladite feuille (10) sur toute la périphérie de l'extrémité proximale (11) ;
- ladite extrémité distale (12) se prolonge par une douille solidarisé, de manière étanche, aux plis couchés et enroulés de ladite feuille (10) sur toute la périphérie de l'extrémité distale (12) dudit implant (1) ;
- ladite feuille (10) est déformable plastiquement pour permettre l'expansion de l'implant de la configuration repliée à la configuration déployée en formant une enveloppe étanche enfermant l'implant et permettant d'éviter les fuites lors de l'injection d'un fluide à l'intérieur de l'implant (1) et l'enveloppe.

Dans certains modes de réalisation, l'un des implants sélectionnables est un implant (1) osseux expansible de chirurgie orthopédique vétérinaire pour la restauration de volume et/ou géométrie d'un os, par une expansion entre une configuration repliée et une configuration déployée, ledit implant comportant un axe central (3) et s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces, par exemple supérieure et inférieure, de l'implant comportant chacune au moins un plateau (13, 14, 15) pour le contact avec les tissus osseux, chacun des plateaux étant supportés par au moins deux bras de support (131, 141, 151) chacun, par l'intermédiaire d'une charnière sur l'axe central (3) et d'une charnière sous le plateau (13, 14, 15) respectif de chacun desdits bras de support (131, 141, 151), caractérisé en ce que :
- une douille ou bague d'expansion (20) disposée dans le même axe que ledit axe central (3) ;
- au moins deux bras d'expansion (132, 142, 152) comportent chacun une charnière les reliant à l'une des extrémités de l'un des plateaux (13, 14, 15) et une charnière les reliant à ladite douille ou bague d'expansion (20) ;
- ladite douille ou bague d'expansion (20) et l'extrémité proximale de l'axe central (3) sont aptes à coopérer, respectivement ou inversement, avec un tube creux (A1) de préhension de l'implant (1) d'un instrument d'implantation (A) et avec une tige d'expansion (A3) dudit instrument (A) ;
- ladite tige d'expansion (A3) est apte à coulisser à l'intérieur dudit tube creux (A1), de façon à permettre de provoquer un éloignement entre ladite douille (3) et ledit axe central (3), exerçant une traction sur les plateaux (13, 14, 15), par l'intermédiaire des bras d'expansion (132, 142, 152), ce qui engendre un pivotement desdits bras de support (131, 141, 151) provoquant l'éloignement des plateaux (13, 14, 15) par rapport à l'axe central (3), de manière à résulter en une expansion contrôlée de l'implant (1) entre ladite configuration repliée et ladite configuration déployée.

Dans certains de ces modes de réalisation, l'implant (1) comporte une enveloppe renfermant ledit implant depuis l'extrémité proximale (11) jusqu'à l'extrémité distale (12) et en ce que :
- ladite enveloppe est formée par une feuille (10) en alliage biocompatible de métaux, refermée sur elle-même de manière étanche ;
- ladite feuille (10) présente, au moins dans la configuration repliée, une pluralité de paires de plis, chacune des paires comportant un pli antiforme (101), dit convexe, et un pli synforme (102), dit concave, lesdits plis étant couchés les uns sur les autres en configuration repliée de sorte que les surfaces présentes entre chacun desdits plis convexes et concaves soient enroulées autour de l'axe longitudinal (L) ;
- ladite extrémité proximale (11) se prolonge par un manchon d'étanchéité solidarisé de manière étanche, aux plis couchés et enroulés de ladite feuille (10) sur toute la périphérie de l'extrémité proximale (11) ;
- ladite extrémité distale (12) se prolonge par une douille solidarisé, de manière étanche, aux plis couchés et enroulés de ladite feuille (10) sur toute la périphérie de l'extrémité distale (12) dudit implant (1) ;
- ladite feuille (10) est déformable plastiquement pour permettre l'expansion de l'implant de la configuration repliée à la configuration déployée en formant une enveloppe étanche enfermant l'implant et permettant d'éviter les fuites lors de l'injection d'un fluide à l'intérieur de l'implant (1) et l'enveloppe.

La présente demande concerne également une méthode de planification d'implantation de système d'implants selon l'une des revendications précédentes, pour la restauration de volume et/ou géométrie d'un os, par une expansion entre une configuration repliée et une configuration déployée, caractérisé en ce qu'elle comporte une sélection, automatique et/ou par le praticien réalisant l'implantation, d'au moins un type de fracture identifié dans au moins une classification standardisée, via des moyens informatiques (PC) et présenté audit praticien, puis l'accès, suite à cette sélection, à au moins un système d'implants compatible avec ledit type de fracture et à au moins un protocole opératoire comprenant une succession d'étapes à réaliser en fonction de ladite sélection.

Dans certains modes de réalisation, la méthode est mise en oeuvre par des moyens informatiques exécutant des instructions sur un processeur permettant :
- l'affichage d'informations relatives à au moins une classification des types de fractures identifiées en traumatologie ;
- la sélection d'au moins un type de fracture et d'au moins une information relative aux dimensions de l'os fracturé, puis
- l'affichage de consignes ou conseils sur la procédure à suivre et sur les divers systèmes à utiliser pour la procédure, par exemple avec un affichage de références des implants ou des divers systèmes utilisables pour chaque fracture identifiée dans la classification, puis
- la sélection d'un choix parmi ces conseils, provoquant l'affichage d'instructions ou conseils suivants pour fournir ladite assistance étape par étape.

Dans certains modes de réalisation, l'affichage de consignes ou conseils comprend l'affichage de fonctions sélectionnables pour réaliser un choix parmi des actes à effectuer pour continuer la procédure et/ou parmi des implants à sélectionner en fonction du protocole opératoire choisi en fonction du type de fracture identifié d'après ladite classification, permettant au praticien de valider un choix et éventuellement proposer des options prédéfinies dans le protocole validé, comme par exemple l'acquisition de radiographies à la fin de certaines étapes mises en oeuvre.

Dans certains modes de réalisation, lesdites fonctions sélectionnables incluent des fonctions permettant au praticien de modifier ledit protocole et générer l'affichage d'une fenêtre de modification du protocole, soit pour une sélection d'un protocole parmi d'autres protocoles possibles pour ledit type de fracture identifié d'après ladite classification, soit pour la création d'un protocole opératoire personnalisé pour la fracture en cours de réparation.

Dans certains modes de réalisation, lesdites fonctions sélectionnables permettent un enregistrement des opérations et protocoles opératoires, notamment personnalisés, utilisés par les différents praticiens et leurs associations aux fractures, avec un éventuel enregistrement de protocoles personnalisés créées par les praticiens, afin de les proposer ultérieurement.

Dans certains modes de réalisation, la méthode comporte :
Une Détection de fracture par système d'identification par intelligence artificielle, gra^ce à des Radios/ Scanner / IRM chargés dans le logiciel et par reconnaissance de défaut et/ou de différence avec la supposé réalité, la zone supposée fracturée est identifiée, pour faciliter l'identification d'une singularité pour le praticien. La base de donnée est enrichie au fur et à mesure en tenant compte des choix du praticien.

Certains modes de réalisation prévoient une reconnaissance de la typologie de fracture avec identification selon la Classification AO (association orthopédique) ou toute autre classification enregistrée dans les moyens informatiques (PC). Le logiciel peut alors classer le type, l'importance, et la position de la fracture, définis par exemple par :
- la Zone : Distale / Médiane / Proximale, décrite comme cunéiforme, Biconcave, ou en galette
- l'importance : Vertèbre normale / Fracture légère / fracture Modérée / Fracture sévère.

De préférence, une osthéodensitométrie est alors pratiquée afin que le logiciel puisse analyser et comparer avec un modèle afin d'établir les risque direct et futurs de la fracture.

D'autre part Basé sur l'identification et le niveau de fracture selon la réduction à effectuer, le logiciel peut proposer le type d'implant et de protocole le mieux adapté à la morphologie / à la densité osseuse, au type, et importance de la fracture. L'implant le mieux adapté est choisi en général en fonction du type de correction: et de préférence, le logiciel propose, mais le praticien doit valider / et / ou peut modifier la proposition du logiciel.

Certains modes de réalisation prévoient une assistance pendant l'opération avec contrôle de conformité du protocole choisi par le praticien et/ou corrigé par le praticien, avec une augmentation et/ou une réduction du volume corrigé.

D'autre part, dans certains modes de réalisation, le système comporte au moins un instrument (A) s'étendant selon un axe longitudinal (L) entre une extrémité proximale (11) apte à coopérer avec ledit instrument d'implantation (A) pour tenir l'implant et une extrémité distale (12) destinée à être insérée en premier dans l'os, ledit instrument (A) comportant une partie principale préhensible par un praticien, une portion (AA) porte-implant comprenant un tube (A0) de préhension s'étendant selon l'axe longitudinal (L) et possédant, à son extrémité distale, des moyens de retenue complémentaires de moyens de coopération de l'implant, pour tenir l'implant par son extrémité proximale (11), ledit instrument (A) étant caractérisé en ce qu'il comporte un instrument (Ac) d'injection de fluide, tel que du ciment osseux, dans ledit implant (1) comprenant au moins une cavité apte à recevoir le fluide, ledit instrument d'injection (Ac) étant disposé en arrière de la portion porte-implant (AA) le long de l'axe longitudinal (L) et comprenant au moins une canule (A0, A1, A3) pour acheminer le fluide jusqu'à l'intérieur de l'implant (1) alors qu'il est encore tenu par ladite portion porte-implant (AA).

Dans certains modes de réalisation, l'instrument (A) comporte également un instrument (Ae) d'expansion de l'implant (1) apte à coopérer avec des moyens d'expansion mécaniques de l'implant (1), grâce à une tige d'expansion (A3) traversant ladite portion porte-implant (AA) et ledit tube (A0) de préhension pour actionner lesdits moyens d'expansion mécaniques. Dans certains de ces modes de réalisation, ladite tige d'expansion (A3) traverse ledit instrument d'injection (Ac) jusqu'à l'extrémité distale du tube (A0) de préhension.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un autre mode de réalisation à moins que l'inverse ne soit explicitement mentionné ou qu'il ne soit évident que ces caractéristiques sont incompatibles ou que la combinaison ne fournisse pas une solution à au moins un des problèmes techniques mentionnés dans la présente demande. De plus, les caractéristiques techniques décrites dans un mode de réalisation donné peuvent être isolées des autres caractéristiques de ce mode à moins que l'inverse ne soit explicitement mentionné.

Liste détaillée des références dans les figures :
- 1: implant
- 10: feuille
- 11: extrémité proximale
- 101: pli antiforme
- 102: pli synforme
- 110: soudure proximale
- 12: extrémité distale
- 120: soudure distale (liaison étanche)
- 121: fixation par compression (e.g., bague fendue)
- 3: axe central
- 31: conduit dans l'axe central
- 32: ouvertures du conduit de l'axe central
- 13: premier plateau
- 14: deuxième plateau
- 15: troisième plateau
- 20: bague d'expansion
- 131: bras support du premier plateau
- 141: bras support du deuxième plateau
- 151: bras support du troisième plateau
- 132: bras d'expansion du premier plateau
- 142: bras d'expansion du deuxième plateau
- 152: bras d'expansion du troisième plateau
- 130: bras support central du premier plateau
- 140: bras support central du deuxième plateau
- 150: bras support central du troisième plateau
- A: instrument d'implantation
- Ac: instrument d'injection de fluide
- A0: tube creux de préhension
- A1: canule d'injection
- A3: tige d'expansion
- V: vertèbre
- VCF: Fracture de compression vertébrale

## Revendications

1. Système de chirurgie orthopédique vétérinaire, pour la restauration de volume et/ou géométrie d'un os, par une expansion entre une configuration repliée et une configuration déployée d'au moins un implant (1) osseux expansible comportant un corps s'étendant selon un axe longitudinal (L) entre une extrémité proximale (11) apte à coopérer avec un instrument d'implantation (A) pour tenir l'implant et une extrémité distale (12) destinée à être insérée en premier dans l'os, le système étant **caractérisé en ce qu'**il comprend au moins un implant (1) osseux expansible et au moins un instrument d'implantation (A) correspondant, sélectionnés parmi une pluralité d'implants (1) et d'instruments (A) disponibles grâce à des moyens informatiques (PC) comprenant une interface homme-machine et exécutant des instructions sur un processeur permettant :
- l'affichage d'informations relatives à au moins une classification standardisée des divers types de fractures identifiés, notamment les fractures de compression vertébrale ;
- la sélection d'au moins un type de fracture et d'au moins une information relative aux dimensions de l'os fracturé, puis
- l'affichage des implants (1) et instruments (A) du système qui sont utilisables pour la restauration envisagée et éventuellement de consignes ou conseils sur la procédure à suivre, par exemple avec un affichage de références des implants ou des divers systèmes utilisables pour chaque fracture identifiée dans la classification, puis
- la sélection d'un choix parmi ces propositions et conseils, par l'utilisateur via l'interface homme-machine, provoquant l'affichage d'instructions ou conseils suivants pour fournir une assistance à l'intervention ou sa planification, avec l'acquisition de données fournies par l'utilisateur sur l'intervention chirurgicale envisagée.

2. Système selon la revendication 1, **caractérisé en ce que** ladite sélection du type de fracture dans la classification est réalisée soit par l'utilisateur soit de manière automatique par les moyens informatiques (PC) grâce à un entrainement sur des bases de données de fractures pour leur classification automatique à partir d'informations techniques et/ou d'images fournies par l'utilisateur et/ou acquises au préalable par les moyens informatique, puis leur mise en relation avec ladite classification standardisée des fractures, la sélection automatique étant de préférence validable ou modifiable par l'utilisateur.

3. Système selon l'une des revendications précédentes, **caractérisé en ce que** l'un des implants sélectionnables est un implant (1) osseux expansible de chirurgie orthopédique vétérinaire pour la restauration de volume et/ou géométrie d'un os, par une expansion entre une configuration repliée et une configuration déployée, ledit implant comportant un corps creux s'étendant selon un axe longitudinal (L) entre une extrémité proximale (11) apte à coopérer avec un instrument d'implantation (A) pour tenir l'implant et une extrémité distale (12) destinée à être insérée en premier dans l'os, et qui est **caractérisé en ce que** :
- la paroi dudit corps creux est formée par une feuille (10) en alliage biocompatible de métaux, refermée sur elle-même de manière étanche, entre lesdites extrémités proximale (11) et distale (12) ;
- ladite feuille (10) présente, au moins dans la configuration repliée, une pluralité de paires de plis, chacune des paires comportant un pli antiforme (101), dit convexe, et un pli synforme (102), dit concave, lesdits plis étant couchés les uns sur les autres en configuration repliée de sorte que les surfaces présentes entre chacun desdits plis convexe et concave soient enroulées autour de l'axe longitudinal (L),
- ladite extrémité proximale (11) comporte une bague solidarisée, de manière étanche, aux plis couchés et enroulés de ladite feuille (10) sur toute la périphérie de l'extrémité distale (11), l'ouverture traversant la bague fournissant une entrée vers l'intérieur du corps creux de l'implant (1),
- ladite extrémité distale (12) comporte un culot fermant l'extrémité distale (12) et solidarisé, de manière étanche, aux plis couchés et enroulés de ladite feuille (10) sur toute la périphérie de l'extrémité distale dudit corps creux
- ladite feuille (10) étant déformable plastiquement pour permettre l'expansion de l'implant de la configuration repliée à la configuration déployée, lors de l'injection d'un fluide à l'intérieur de l'implant (1).

4. Système selon l'une des revendications précédentes, **caractérisé en ce que** l'un des implants sélectionnables est un implant (1) osseux expansible de chirurgie orthopédique vétérinaire pour la restauration de volume et/ou géométrie d'un os, par une expansion entre une configuration repliée et une configuration déployée, ledit implant s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces de l'implant, par exemple supérieure et inférieure, comportant chacune un plateau (13, 14) pour le contact avec les tissus osseux, chacun des plateaux comportant une portion centrale (130, 140) reliée, par l'intermédiaire d'au moins une charnière, à au moins une paire de bras de support (131, 141) orientés chacun dans des directions opposées au sein de chaque paire, un bras de chaque paire étant relié par une charnière à l'extrémité distale (11) tandis que l'autre bras est relié par une charnière à l'extrémité proximale (12), l'implant (1) étant apte à recevoir ou comportant un axe (3) central s'étendant au travers d'un manchon de coulissement à l'extrémité proximale (11) jusqu'à une bague ou une douille de traction à l'extrémité distale (12) où il est apte à transmettre une traction, lors de son actionnement par un instrument (A), sur l'extrémité distale (12) pour permettre de la rapprocher de l'extrémité proximale (11), en engendrant le pivotement des bras de support (131, 141) provoquant l'éloignement des plateaux (13, 14) l'un par rapport à l'autre et, par conséquent, l'expansion de l'implant entre la configuration repliée et la configuration déployée.

5. Système selon la revendication 4, **caractérisé en ce que** l'implant (1) est également **caractérisé en ce que** :
- au moins deux autres faces de l'implant, entre celles comportant les plateaux, sont couvertes d'au moins une feuille (10) par face, en alliage biocompatible de métaux, et solidarisée de manière étanche aux portions centrales (130, 140) sous les plateaux, à des faces latérales des bras (131, 141) et à des faces latérales de l'extrémité proximale (11) et de l'extrémité distale (12),
- ladite feuille (10) est déformable plastiquement pour permettre l'expansion de l'implant et présente, au moins dans la configuration repliée, une pluralité de plis antiformes (101), dit convexes, et synformes (102), dit concaves, lesdits plis étant couchés les uns sur les autres en configuration repliée, la surface totale de ladite feuille étant supérieure ou égale à la surface latérale de l'implant en configuration déployée de façon à former un compartiment étanche apte à accueillir un fluide à l'intérieur de la cavité obtenue par l'expansion de l'implant.

6. Système selon l'une des revendications 4 et 5, **caractérisé en ce que** ledit implant (1) comporte une enveloppe renfermant ledit implant depuis l'extrémité proximale (11) jusqu'à l'extrémité distale (12) et **en ce que** :
- ladite enveloppe est formée par une feuille (10) en alliage biocompatible de métaux, refermée sur elle-même de manière étanche ;
- ladite feuille (10) présente, au moins dans la configuration repliée, une pluralité de paires de plis, chacune des paires comportant un pli antiforme (101), dit convexe, et un pli synforme (102), dit concave, lesdits plis étant couchés les uns sur les autres en configuration repliée de sorte que les surfaces présentes entre chacun desdits plis convexes et concaves soient enroulées autour de l'axe longitudinal (L) ;
- ladite extrémité proximale (11) se prolonge par un manchon d'étanchéité solidarisé de manière étanche, aux plis couchés et enroulés de ladite feuille (10) sur toute la périphérie de l'extrémité proximale (11) ;
- ladite extrémité distale (12) se prolonge par une douille solidarisé, de manière étanche, aux plis couchés et enroulés de ladite feuille (10) sur toute la périphérie de l'extrémité distale (12) dudit implant (1) ;
- ladite feuille (10) est déformable plastiquement pour permettre l'expansion de l'implant de la configuration repliée à la configuration déployée en formant une enveloppe étanche enfermant l'implant et permettant d'éviter les fuites lors de l'injection d'un fluide à l'intérieur de l'implant (1) et l'enveloppe.

7. Système selon l'une des revendications précédentes, **caractérisé en ce que** l'un des implants sélectionnables est un implant (1) osseux expansible de chirurgie orthopédique vétérinaire pour la restauration de volume et/ou géométrie d'un os, par une expansion entre une configuration repliée et une configuration déployée, ledit implant comportant un axe central (3) et s'étendant selon un axe longitudinal (L) entre une extrémité proximale (11) apte à coopérer avec un instrument d'implantation (A) pour tenir l'implant et une extrémité distale (12) destinée à être insérée en premier dans l'os, au moins deux faces, par exemple supérieure et inférieure, de l'implant comportant chacune au moins un plateau (13, 14, 15) pour le contact avec les tissus osseux, chacun des plateaux étant supportés par au moins deux bras de support (131, 141, 151) chacun, par l'intermédiaire d'une charnière sur l'axe central (3) et d'une charnière sous le plateau (13, 14, 15) respectif de chacun desdits bras de support (131, 141, 151), **caractérisé en ce que** :
- une douille ou bague d'expansion (20) disposée dans le même axe que ledit axe central (3) ;
- au moins deux bras d'expansion (132, 142, 152) comportent chacun une charnière les reliant à l'une des extrémités de l'un des plateaux (13, 14, 15) et une charnière les reliant à ladite douille ou bague d'expansion (20) ;
- ladite douille ou bague d'expansion (20) et l'extrémité proximale de l'axe central (3) sont aptes à coopérer, respectivement ou inversement, avec un tube creux (A1) de préhension de l'implant (1) d'un instrument d'implantation (A) et avec une tige d'expansion (A3) dudit instrument (A) ;
- ladite tige d'expansion (A3) est apte à coulisser à l'intérieur dudit tube creux (A1), de façon à permettre de provoquer un éloignement entre ladite douille (3) et ledit axe central (3), exerçant une traction sur les plateaux (13, 14, 15), par l'intermédiaire des bras d'expansion (132, 142, 152), ce qui engendre un pivotement desdits bras de support (131, 141, 151) provoquant l'éloignement des plateaux (13, 14, 15) par rapport à l'axe central (3), de manière à résulter en une expansion contrôlée de l'implant (1) entre ladite configuration repliée et ladite configuration déployée.

8. Système selon la revendication 7, **caractérisé en ce que** l'implant (1) comporte une enveloppe renfermant ledit implant depuis l'extrémité proximale (11) jusqu'à l'extrémité distale (12) et **en ce que** :
- ladite enveloppe est formée par une feuille (10) en alliage biocompatible de métaux, refermée sur elle-même de manière étanche ;
- ladite feuille (10) présente, au moins dans la configuration repliée, une pluralité de paires de plis, chacune des paires comportant un pli antiforme (101), dit convexe, et un pli synforme (102), dit concave, lesdits plis étant couchés les uns sur les autres en configuration repliée de sorte que les surfaces présentes entre chacun desdits plis convexes et concaves soient enroulées autour de l'axe longitudinal (L) ;
- ladite extrémité proximale (11) se prolonge par un manchon d'étanchéité solidarisé de manière étanche, aux plis couchés et enroulés de ladite feuille (10) sur toute la périphérie de l'extrémité proximale (11) ;
- ladite extrémité distale (12) se prolonge par une douille solidarisé, de manière étanche, aux plis couchés et enroulés de ladite feuille (10) sur toute la périphérie de l'extrémité distale (12) dudit implant (1) ;
- ladite feuille (10) est déformable plastiquement pour permettre l'expansion de l'implant de la configuration repliée à la configuration déployée en formant une enveloppe étanche enfermant l'implant et permettant d'éviter les fuites lors de l'injection d'un fluide à l'intérieur de l'implant (1) et l'enveloppe.

9. Méthode de planification d'implantation de système d'implants selon l'une des revendications précédentes, pour la restauration de volume et/ou géométrie d'un os, par une expansion entre une configuration repliée et une configuration déployée, **caractérisé en ce qu'**elle comporte une sélection, automatique et/ou par le praticien réalisant l'implantation, d'au moins un type de fracture identifié dans au moins une classification standardisée, via des moyens informatiques (PC) et présenté audit praticien, puis l'accès, suite à cette sélection, à au moins un système d'implants compatible avec ledit type de fracture et à au moins un protocole opératoire comprenant une succession d'étapes à réaliser en fonction de ladite sélection.

10. Méthode de planification selon la revendication 9, **caractérisée en ce qu'**elle est mise en oeuvre par des moyens informatiques exécutant des instructions sur un processeur permettant :
- l'affichage d'informations relatives à au moins une classification des types de fractures identifiées en traumatologie ;
- la sélection d'au moins un type de fracture et d'au moins une information relative aux dimensions de l'os fracturé, puis
- l'affichage de consignes ou conseils sur la procédure à suivre et sur les divers systèmes à utiliser pour la procédure, par exemple avec un affichage de références des implants ou des divers systèmes utilisables pour chaque fracture identifiée dans la classification, puis
- la sélection d'un choix parmi ces conseils, provoquant l'affichage d'instructions ou conseils suivants pour fournir ladite assistance étape par étape.

11. Méthode de planification selon la revendication 10, **caractérisée en ce que** l'affichage de consignes ou conseils comprend l'affichage de fonctions sélectionnables pour réaliser un choix parmi des actes à effectuer pour continuer la procédure et/ou parmi des implants à sélectionner en fonction du protocole opératoire choisi en fonction du type de fracture identifié d'après ladite classification, permettant au praticien de valider un choix et éventuellement proposer des options prédéfinies dans le protocole validé, comme par exemple l'acquisition de radiographies à la fin de certaines étapes mises en oeuvre.

12. Méthode de planification selon la revendication 11, **caractérisée en ce que** lesdites fonctions sélectionnables incluent des fonctions permettant au praticien de modifier ledit protocole et générer l'affichage d'une fenêtre de modification du protocole, soit pour une sélection d'un protocole parmi d'autres protocoles possibles pour ledit type de fracture identifié d'après ladite classification, soit pour la création d'un protocole opératoire personnalisé pour la fracture en cours de réparation.

13. Méthode de planification selon la revendication 12, **caractérisée en ce que** lesdites fonctions sélectionnables permettent un enregistrement des opérations et protocoles opératoires, notamment personnalisés, utilisés par les différents praticiens et leurs associations aux fractures, avec un éventuel enregistrement de protocoles personnalisés créées par les praticiens, afin de les proposer ultérieurement.
